# EUROPÄISCHE PATENTANMELDUNG

(11) **EP 4 458 283 A1**
(43) Veröffentlichungstag der Anmeldung: **06.11.2024**
(21) Anmeldenummer: 24020107.9
(22) Anmeldetag: 08.04.2024
(51) Int. Cl.: A61B 17/22, A61H 23/00

(54) **VORRICHTUNG ZUR UMLIENKUNG UND FOKUSSIERUNG VON STOSSWELLEN; VERWENDUNG EINER VORRICHTUNG ZUR UMLENKUNG UND FOKUSSIERUNG VON STOSSWELLEN; VERFAHREN ZUM FOKUSSIEREN UND UMLENKEN EINER STOSSWELLE**

(30) Priorität: 03.05.2023 DE 102023111367
(71) Anmelder: Likamed GmbH, 75031 Eppingen (DE)
(72) Erfinder: NOVER, Konrad, 75031 Eppingen (DE)
(74) Vertreter: Patentanwälte Schuster, Müller & Partner mbB

(57) **Zusammenfassung**

Es werden eine Vorrichtung (1) zur Umlenkung und Fokussierung von Stoßwellen, eine Verwendung einer Vorrichtung (1) zur Umlenkung und Fokussierung von Stoßwellen und ein Verfahren zum Fokussieren und Umlenken einer Stoßwelle vorgeschlagen, wobei die Vorrichtung (1) mindestens einen Applikator (2), wobei mindestens ein Applikator (2) mindestens eine Applikationsfläche (8) aufweist und von mindestens einer Applikationsfläche (8) in einer Stoßrichtung eine Stoßwelle emittiert werden kann, und mindestens einen Reflektor (3), wobei mindestens ein Reflektor (3) mindestens eine Reflexionsfläche (9), und einen Fokuspunkt (11), auf den Stoßwellen durch die Reflexionsflächen (9) fokussiert werden können, aufweist, umfasst, wobei der Fokuspunkt (11) nicht in einer Stoßrichtung liegt, wodurch insbesondere ein Auftreffen der primären Stoßwelle auf das zu behandelnde Gewebe vermieden werden kann.

## Beschreibung

### Stand der Technik

Die Erfindung geht aus von einer Vorrichtung zur Umlenkung und Fokussierung von Stoßwellen, nach der Gattung des Anspruchs 1, einer Verwendung einer Vorrichtung zur Umlenkung und Fokussierung von Stoßwellen, nach der Gattung des Anspruchs 8, und einem Verfahren zum Fokussieren und Umlenken einer Stoßwelle, nach der Gattung des Anspruchs 10.

Chronische schmerzhafte Erkrankungen der Weichteile des menschlichen Bewegungsapparates sind sehr häufig und der Patient leidet oft monatelang ohne erkennbare Verbesserung und stellen nicht nur eine Belastung für den Patienten dar, sondern aufgrund der häufig langwierigen Behandlung auch für das Gesundheitssystem. Je nachdem, ob der Muskel und die Faszien oder die Sehnenansätze betroffen sind, unterscheidet man myofasziale Schmerzsyndrome und Tendinopathien. Sie betreffen die Muskeln und seine Hüllen (Faszien) sowie die Sehnen an den Knochenansätzen. Die Schmerzen resultieren zumeist aus Fehl- und Überbeanspruchung und sind durch Steifigkeit und Verkürzungen der Muskeln sowie durch lokale Verhärtungen (Myogelosen) gekennzeichnet, die im Sinne von so genannten "Trigger Points" sehr häufig abnorm druckschmerzhaft sind. Auch entzündliche Prozesse spielen eine Rolle und es kann zu Kalkablagerungen an den Knochenansatzregionen der Sehnen kommen. Die pathophysiologischen Mechanismen sind sehr komplex und noch nicht vollständig verstanden. Typische Krankheitsbilder sind die adhäsive capsulitis und tendinosis calcarea der Schulter, auch Kalkschulter ("frozen shoulder"), genannt und die Plantarfasciitis des Fußes, die, wenn sie mit Kalkablagerungen und knöchernen Vorwölbungen der Ferse einhergeht, Fersensporn genannt wird. Die extrakorporale Stoßwellentherapie (ESWT) hat sich als nicht-invasive Behandlung dieser Krankheitsbilder bewährt. Ihre Wirksamkeit konnte in kontrollierten Studien belegt werden, etwa bei der Kalkschulter (Farr, S. et al., Knee Surgery, Sports Traumatology, Arthroscopy 2011, 19 12; Abo Al-Khair, M. A. et al., The Physician and Sportsmedicine 2021, 49, 4) und dem Fersensporn (Berbrayer, D. et al., PM&R 2014, 6 2; Pisani, E. et al., "Effectiveness and tolerability of focal versus radial extracorporeal shock wave." Beyond Rheumatology 2020, 03/2020) sowie bei myofaszialen Schmerzsyndromen des Rückens (Gezglnaslan, Ö. et al., Archives of Rheumatology 2020, 35, 1; Min Ji, H. et al., Annals of Rehabilitation Medicine 2012, 36, 5). Inzwischen ist die Behandlung des Fersensporns keine individuelle Gesundheitsleistung (IGe-Leistung) mehr, sondern die Kosten werden seit Januar 2019 von den gesetzlichen Krankenkassen übernommen. Jedoch ist es schwer, das exakte Behandlungsareal mit dem wenige Millimeter großen Fokuspunkt zu treffen. Durch dieses Problem verlaufen Behandlung, teilweise erfolglos und der Patient muss weiterhin Schmerzen erleiden.

Eine objektive, zielgerichtete Behandlung durch Schmerzmonitoring existiert bislang noch nicht. Diverse Forschungsarbeiten konzentrierten sich auf die Anwendung Laser evozierter Potentiale (LEP) in der Schmerzdiagnostik (zur Übersicht siehe Treede, R. D. et al., Neurophysiologie Clinique/Clinical Neurophysiology 2003, 33, 6). Für das geplante Vorhaben von besonderer Bedeutung sind Anwendungen bei muskuloskelettalen Schmerzen (Lorenz, J., Zeitschrift für Rheumatologie 1998, 57, 2, und Lorenz, J. Pain Forum 1998, 7, 212) und zum Nachweis von Entzündungsprozessen (Domnick, C. et al., Journal of pain research 2009, 2, 49).

Stoßwellen sind energiereiche Wellen, die Wasser und Weichteilgewebe durchdringen können. Die ESWT wurde ursprünglich eingeführt, um Nieren- und Gallensteine ohne Operation zu entfernen, hat aber seit mehr als 20 Jahren einen viel breiteren Anwendungsbereich der Medizin, insbesondere der Orthopädie, erobert. Ein wesentlicher Vorteil einer fokussierten ESWT ist es, dass die Energieflussdichte auf tiefer gelegenes Zielgewebe millimetergenau ausgerichtet werden kann, so dass umliegendes Gewebe geschont wird. Hingegen ist bei einer radialen Stoßwellenquelle die Energieflussdichte an der Hautoberfläche maximal und breitet sich dann radial in die tieferen Gewebeschichten aus. Die Fokussierung der Stoßwellen ist technisch aufwendig und verwendet elektrohydraulische, elektromagnetische und piezoelektrische Verfahren (Gerdesmeyer, L. M. et al., Der Orthopade 2002, 31, 7). Vergleichsweise weniger aufwendig und daher kostengünstiger ist die Erzeugung radialer Stoßwellen auf der Basis eines ballistischen Stoßwellenerzeugungskonzepts, wie es beispielsweise in der EP 4 039 202 B1 beschrieben wird. Dabei wird ein Projektil mit Hilfe komprimierter Luft beschleunigt. Nach einer kurzen Beschleunigungsstrecke wird das Projektil abrupt auf einer Prallfläche gestoppt. Die Prallfläche befindet sich auf einem sogenannten Applikator, welcher die entstehende Stoßwelle auf die Haut des Patienten überträgt. Bislang gibt es noch keine Geräte, die mit dem einfachen ballistischen Funktionsprinzip, wie es zur Erzeugung radialer Stoßwellen verwendet wird, auch fokussierte Stoßwellen generieren können.

Derzeitig gibt es drei verschiedene Verfahren zur Erzeugung von Stoßwellen für eine fokussierte Stoßwellentherapie. Dabei basieren sämtliche Erzeugungsvarianten auf einer elektrischen Energiequelle (Ueberle, F. Einsatz von Stoßwellen in der Medizin. Medizintechnik: Verfahren - Systeme - Informationsverarbeitung, 2017). In dem elektrohydraulischen Prinzip wird durch das Anlegen einer Hochspannung zwischen zwei Elektroden unter Wasser ein energiereicher Funken generiert und die daraus entstehende Druckwelle über einen elliptischen Reflektor an einem zweiten Brennpunkt fokussiert (Wess, O., Journal für Mineralstoffwechsel 2004, 11, 4). Das elektromagnetische Prinzip generiert Stoßwellen durch das Magnetfeld einer stromdurchflossenen Spule und der Auslenkung einer Membran - ähnlich einem Lautsprecher. Durch einen Reflektor oder eine akustische Linse wird das Drucksignal fokussiert (Crevenna, R. et al., Current Physical Medicine and Rehabilitation Reports 2021, 9). Beim piezoelektrischen Prinzip dient die mechanische Ausdehnung mehrerer sphärisch angeordneter Piezoelemente (durch das Anlegen einer Spannung) der Erzeugung der Stoßwellen, welche durch die sphärische Anordnung fokussiert werden. Die radiale Stoßwellentherapie basiert bisher auf dem ballistischen Druckpulsprinzip (Ueberle, F. Einsatz von Stoßwellen in der Medizin. Medizintechnik: Verfahren - Systeme- Informationsverarbeitung, 2017). Im Unterschied zu den fokussierten Prinzipien ist die treibende Kraft des ballistischen Prinzips die Druckluft. In einem kleinen, etwa 15 cm langen Metallrohr wird ein zylindrisches Projektil mit einer pneumatisch erzeugten Druckluft beschleunigt und prallt auf einen Prallkörper am Ende dieses Rohrs. Durch den Zusammenprall entsteht eine hohe Druckwelle, die über den Applikator und unter Verwendung von Ultraschallgel auf die Haut des Patienten übertragen wird. Diese breitet sich radial vom Applikationsort in das Gewebe aus und verliert mit der Eindringtiefe ihre Energie.

Zum heutigen Stand der Technik gibt es verschiedene Möglichkeiten, Wellen zu fokussieren. Für kugelförmige Wellen werden für die Fokussierung häufig Rotationshalbellipsoide verwendet. Die Wellen werden im Inneren des Ellipsoids erzeugt und an ihren Wänden reflektiert. Durch die Form des Ellipsoids kann bestimmt werden, in welcher Entfernung sich die reflektierten Wellen überlagern und somit den Fokus bilden (Rassweiler, J. et al., Extrakorporale Stoßwellentherapie der Urolithiasis. SLK-Kliniken Heilbronn GmbH, Urologie, Heilbronn, Deutschland). Eine weitere Art, Wellen zu fokussieren, ist der Einsatz von akustischen Linsen. Dabei finden sowohl konkave als auch konvexe Linsenformen ihre Anwendung. Der Durchtritt der Wellen durch die Linse führt dazu, dass diese gekrümmt werden und an einem Fokuspunkt hinter der Linse sich treffen. Der Fokuspunkt ist hier wiederum durch die Linsenform und das Linsenmaterial einstellbar. Eine etwas neuere Methode ist die Fokussierung akustischer Druckwellen durch akustische Metamaterialien (Löfken, J.O., Akustische Linse aus ungewöhnlichem Material. 15. September 2021., www.weltderphysik.de, Abruf: 25.03.2024). Diese bestehen aus einer symmetrischen beziehungsweise spezifischen Anordnung von Strukturen. Je nach Frequenz der Welle und Wahl des Materials wird die Welle unterschiedlich reflektiert bzw. gestreut. Wenn die Frequenz der Welle nahe der Resonanzfrequenz des Strukturmaterials liegt, wird die Schallwelle vielfach gestreut und die Summe der gestreuten Wellen ergibt einen negativen Brechungsindex. Die negative Wellenbrechung sorgt dann für eine Fokussierung der Wellen. Bisher wurde in diesem Bereich der Fokussierung noch wenig geforscht, man geht aber von einem hohen Potential dieser Methode für vielerlei Anwendungen sowohl in der Medizin als auch in der Industrie aus.

Der Erfindung liegt daher die Aufgabe zugrunde, eine die Nachteile des Standes der Technik überwindende Vorrichtung zur Umlenkung und Fokussierung von Stoßwellen, eine die Nachteile des Standes der Technik überwindende Verwendung einer Vorrichtung zur Umlenkung und Fokussierung von Stoßwellen und ein die Nachteile des Standes der Technik überwindendes Verfahren zum Fokussieren und Umlenken einer Stoßwelle bereitzustellen.

### Die Erfindung und ihre Vorteile

Die erfindungsgemäße Vorrichtung zur Umlenkung und Fokussierung von Stoßwellen, mit den Merkmalen des Anspruchs 1, die erfindungsgemäße Verwendung einer Vorrichtung zur Umlenkung und Fokussierung von Stoßwellen, mit den Merkmalen des Anspruchs 8, und das erfindungsgemäße Verfahren zum Fokussieren und Umlenken einer Stoßwelle, mit den Merkmalen des Anspruchs 10, haben demgegenüber den Vorteil, dass die Vorrichtung mindestens einen Applikator, wobei mindestens ein Applikator mindestens eine Applikationsfläche aufweist und von mindestens einer Applikationsfläche in einer Stoßrichtung eine Stoßwelle emittiert werden kann, und mindestens einen Reflektor, wobei mindestens ein Reflektor mindestens eine Reflexionsfläche, und einen Fokuspunkt, auf den Stoßwellen durch die Reflexionsflächen fokussiert werden können, aufweist, umfasst, wobei der Fokuspunkt nicht in einer Stoßrichtung liegt, wodurch insbesondere ein Auftreffen der primären Stoßwelle auf das zu behandelnde Gewebe vermieden werden kann. Dies erlaubt es gezielter die Intensität an der Behandlungsstelle einzustellen, wodurch unnötige Schmerzen und das Auftreten von Verletzungen, wie beispielsweise Hämatomen, durch die Behandlung des Patienten gemindert werden können. Der Fokuspunkt liegt vorzugsweise schräg zur Stoßrichtung des Applikators (näherungsweise tangential) in Richtung einer Reflexionsfläche. Die Applikationsfläche der Stoßwellen zeigt ebenfalls in Richtung der Reflexionsfläche. Dadurch, dass Stoß und Applikationsfläche in Richtung der Reflexionsfläche zeigen, wird der größte Teil der Stoßwelle reflektiert und fokussiert und nur ein minimaler Anteil dringt unfokussiert in das umliegende Medium. Durch die Reflexionsfläche werden die sich ausbreitenden Stoßwellen in Richtung des Fokuspunktes umgelenkt und fokussiert. Denkbar wäre es, dass auch eine mehrfache Umlenkung, insbesondere eine doppelte Umlenkung erfolgt, um den Anteil der unfokussiert abgegebenen Stoßwelle weiter zu minimieren.

Nach einer vorteilhaften Ausgestaltung der erfindungsgemäßen Vorrichtung sind mindestens ein Applikator und mindestens ein Reflektor reversibel miteinander verbunden. Dies erlaubt es beispielsweise unterschiedliche Reflektoren zu verwenden. Denkbar wäre es, dass die Reflektoren sich durch unterschiedliche Fokuspunkte und damit unterschiedliche Eindringtiefen, Spotgrößen und dergleichen unterscheiden. Weiterhin wäre es denkbar, dass die Verbindung des Reflektors mit dem Applikator indirekt beispielsweise über das Gehäuse der Vorrichtung oder über eine Verbindung mit einem Projektil-Lauf erfolgt. Diesbezüglich denkbar wäre es, wenn die Verbindung durch Schrauben, eine Klemmverbindung, einen Steckmechanismus, eine Magnetkupplung, Aufschieben auf eine Schiene oder dergleichen erfolgt. Denkbar wäre es, dass der Reflektor sich auf einem Handstück zusammen mit dem Applikator dem Beschleunigungsrohr für ein Projektil und dem Keramikprojektil montieren lässt.

Nach einer zusätzlichen vorteilhaften Ausgestaltung der erfindungsgemäßen Vorrichtung ist der mindestens eine Reflektor in Form einer Kugel geformt. Die mindestens eine Reflexionsfläche des Reflektors beginnt vorzugsweise auf der Höhe des Applikators und schließt sich um diesen herum, mit einer Öffnung in Richtung des Fokuspunktes. Denkbar wäre es, dass die Öffnung mittels einer Blende verkleinert werden kann.

Nach einer zusätzlichen vorteilhaften Ausgestaltung der erfindungsgemäßen Vorrichtung ist die mindestens eine Reflexionsfläche des mindestens einen Reflektors derart geformt, dass zumindest für einen Teil der Stoßwelle bzw. einen Stoßwellenanteil eine Strecke, die sich als Summe aus einem Streckenanteil A und einem Streckenanteil B ergibt, gleich ist, wobei der Streckenanteil A die Strecke zwischen einem Punkt der Applikationsfläche, von dem dieser Stoßwellenanteil emittiert wird, und einem Punkt auf der Reflexionsfläche, von dem er anschließend reflektiert wird, umfasst, und der Streckenanteil B die Strecke zwischen dem Punkt auf der Reflexionsfläche, von dem dieser Stoßwellenanteil reflektiert wird, und dem Fokuspunkt umfasst. Dabei kann in Fällen der Mehrfachreflektion der Streckenanteil B auch in mehrere Unteranteile (B',B",...) aufgeteilt sein. Dies hat den Vorteil, dass die Stoßwellenanteile vorzugsweise gleichzeitig am Fokuspunkt auftreffen. Stoßwellenanteile sind vektorielle Grö-ßen, die sich aus einer gegebenenfalls veränderlichen Stärke und einer linearen Ausbreitungsrichtung zusammensetzen. Für das Erstellen von Reflexionsfläche wird vorzugsweise ein dreidimensionales Auslegungsverfahren verwendet. Die Punkte des Reflektors werden so ausgelegt, dass die minimale Strecke von der Applikationsfläche zum Reflexionspunkt und dann zum Fokuspunkt für jeden Reflexionspunkt gleich lang ist. Dadurch laufen alle Stoßwellenanteile gleichzeitig in den Fokuspunkt und es kommt zu einer positiven Interferenz. Aus den berechneten Reflexionspunkten wird eine Reflexionsfläche erstellt. Aus der Reflexionsfläche wird der Reflektor erstellt.

Nach einer zusätzlichen vorteilhaften Ausgestaltung der erfindungsgemäßen Vorrichtung weist die Vorrichtung mindestens ein Projektil auf, wobei mindestens ein Projektil aus einer Keramik gefertigt ist und/oder mindestens ein Applikator aus einem Metall und/oder einer Legierung gefertigt ist. Das Material des Applikators zeichnet sich vorzugsweise abgesehen von einer hohen Härte durch eine geringe Schallkennimpedanz, möglichst nahe an der des menschlichen Gewebes, aus. Die geringe Schallkennimpedanz ermöglicht es, einen großen Teil der Stoßwelle aus dem Material des Applikators direkt oder über ein Überbrückungsmaterial in das menschliche Gewebe zu transmittieren.

Nach einer diesbezüglichen vorteilhaften Ausgestaltung der erfindungsgemäßen Vorrichtung ist mindestens ein Applikator aus einer Magnesium- und/oder Aluminiumlegierung gefertigt. Vorzugsweise handelt es sich um eine harte Magnesium- und/oder Aluminiumlegierung. Besonders bevorzugt handelt es sich um eine Aluminiumlegierung mit einer erhöhten Festigkeit, wie beispielsweise eine Aluminiumlegierung nach dem AMS4331A Standard gemäß der American Society of Aeronautic Engineers (SAE).

Der Reflektor kann weiterhin mit einem Übergangsmaterial gefüllt oder mindestens teilweise gefüllt sein, um den transmittierten Anteil der Stoßwelle zu erhöhen, wobei das Übergangsmaterial von der Schallkennimpedanz zwischen Magnesium und dem Menschlichen Gewebe liegt und somit den in das Gewebe transmittierten Teil der Stoßwelle erhöht.

Nach einer zusätzlichen vorteilhaften Ausgestaltung der erfindungsgemäßen Vorrichtung weist mindestens ein Applikator eine kegelförmige Spitze auf. Anders als bei flachen Applikatoren wird der größte Teil der Stoßwelle dadurch nicht nach vorne abgestrahlt, sondern leicht schräg. Durch die Verteilung der Stoßwelle auf der mindestens einen Reflexionsfläche bildet sich beim Eintreten der Stoßwelle vom Reflektor in den Körper ein großer Bereich mit geringer Stoßwellenintensität, welcher sich durch die Fokussierung in einen kleinen Bereich mit hoher Intensität im Fokuspunkt entwickelt. Durch die Verteilung der Stoßwelle mit einer kegelförmigen Applikatorspitze wird somit ein besonders ausgeprägter Kontrast der Stoßwellenintensität zwischen Fokuspunkt und Körperoberfläche erzeugt. Denkbar wäre es, dass der Applikator mittels eines computergestützten Auslegungsverfahrens hergestellt wird.

Die erfindungsgemäße Verwendung einer Vorrichtung zur Umlenkung und Fokussierung von Stoßwellen hat gegenüber dem Stand der Technik den Vorteil, dass, wenn die mittels der erfindungsgemäßen Vorrichtung erzeugten Stoßwellen zur Schmerztherapie eines Patienten eingesetzt werden, die Intensität der Stoßwellen im Fokuspunkt mittels eines Elektroenzephalogramms ermittelt wird, wodurch sich die individuelle Wirkung bemessen lässt.

Nach einer diesbezüglichen vorteilhaften Ausgestaltung der erfindungsgemäßen Verwendung wird die Intensität der Stoßwellen entsprechend eines Signals im Elektroenzephalogramm angepasst. Dies könnte manuell oder automatisch erfolgen. Das pneumatisch-ballistische Prinzip hat einen entscheidenden Vorteil, das darin besteht, die Wirksamkeit der fokussierten extrakorporale Stoßwellentherapie (fESWT) anhand eines neurophysiologischen Schmerzmonitorings zu evaluieren. Bevorzugt werden hirnelektrische Reaktionen (somatosensorisch evozierte Potentiale, SEP) im Elektroenzephalogramm (EEG) durch gepulste fESWT-Stimuli ausgelöst, die als objektive Maße der Schmerzempfindlichkeit in den Zielregionen myofaszialer Schmerzsyndrome herangezogen werden können. Elektrohydraulische, elektromagnetische oder piezoelektrisch erzeugte Stoßwellen, wie sie bislang zur Fokussierung der ESWT verwendet werden, eignen sich nicht für die Untersuchung mit einem EEG, weil die dabei am Applikator entstehenden elektromagnetischen Störfelder erhebliche Artefakte erzeugen. Das erfindungsgemäße Verfahren erzeugt solche Artefakte nicht, da ein ballistisches Stoßwellenerzeugungskonzept verwendet wird. Einige Autoren haben bereits die prinzipielle Anwendbarkeit von fokussiertem Ultraschall zur Objektivierung von Schmerzen aus dem Unterhautgewebe sowie von Muskeln und Gelenkstrukturen gezeigt (Wright, A. et al., Temporal summation of pain from skin, muscle and joint following nocic 2002; Wright, A. et al., Pain 1993, 52, 2; ab Ithel Davies, I. et al., Pain 1996, 67, 1; Legon, W. et al., PloS one 2012, 7, 12; Xu, L. et al., "Measurement of Focused Ultrasound Neural Stimulation; Somatosensory Evoked Poten-tial at Two Separate Skin Temperatures" IEEE International Ultrasonics Symposium (IUS) 2018**).** Die bei diesen Untersuchungen verwendeten Ultraschallreize besitzen allerdings nicht die physikalischen Charakteristika, die für die therapeutische Wirkung von Bedeutung erachtet werden. Fokussierter hochenergetischer Ultraschall erzeugt keinen mit ESWT-Stimuli vergleichbaren mechanischen Stoßwelleneffekt im Zielgewebe, sondern wahrscheinlich durch Dissipation der mechanischen Energie einen kombinierten mechanisch-thermischen Effekt auf Schmerzrezeptoren. Gepulste fESWT-Stimuli besitzen zudem eine sehr steile Anstiegsflanke des mechanischen Impulses. Eine solches Reiz-Zeitprofil ist eine wichtige Voraussetzung, um eine optimale Synchronisation für die reizgetaktete EEG-Mittelwertbildung zu gewährleisten, sodass nach etwa 20 bis 40 Reizwiederholungen somatosensorisch evozierte Potentiale (SEP) im EEG des Patienten erwartet werden können, die ein objektives Korrelat der Schmerzempfindlichkeit in der Zielregion darstellen sollten. Dies führt dazu, dass auf diese Weise das Ausmaß der Schmerzlinderung über wiederholte Sitzungen neurophysiologisch und damit objektiv dokumentiert werden kann. Parallel zu den neurophysiologischen Reaktionen auf die fESWT-Stimuli könnten die subjektiven Empfindungen erfasst werden. Hierzu würden sowohl quantitative Verfahren (Intensitäts- und Schmerzratings) als auch qualitative Verfahren angewendet, die sensorische und affektive Wahrnehmungen differenzieren (Schmerzempfindungsskala, SES). Durch die SES nach Geissner (1988) konnte z.B. gezeigt werden, dass sich die subjektiven Wahrnehmungen von elektrischen Reizen der Faszien, die als schneidend, stechend und zerrend beschrieben werden, von denen der tiefer liegenden Muskeln unterscheiden, die als dumpf, pochend und schlagend beschrieben werden (Schilder et al., 2018). Bei Vorliegen einer Gewebeentzündung dürfte auch eine brennende Empfindung zu erwarten sein. Somit ist es möglich, durch Variation der Eindringtiefe der fESWT-Stimuli die psychophysischen und neurophysiologischen Reaktionen auf Reizungen der Epidermis, der Faszien und der Muskeln zu differenzieren, um die für die Therapie optimal geeignete Eindringtiefe und Reizintensität besser bestimmen zu können. Bei akuter Entzündung der Faszien sollten diese beispielsweise von der mechanischen Irritation weitgehend verschont werden. Ziel ist es, die darunterliegende, durch Verklebung und Verkürzung veränderte Muskulatur durch fokussierte ESWT möglichst selektiv zu stimulieren. Um die zu behandelnde Stelle in der entsprechenden Gewebetiefe mit der richtigen Intensität zu reizen, erscheint daher ein neurophysiologisches Schmerzmonitoring im individuellen Patienten aussichtsreich. In wiederholten Sitzungen kann dadurch zusätzlich der Therapieerfolg dokumentiert werden. Bislang basiert die ESWT-Behandlung auf einer subjektiven Schmerzrückkopplung von dem Patienten an den behandelnden Arzt, um die Energie in die notwendige Zielregion einzubringen.

Das erfindungsgemäße Verfahren zum Fokussieren und Umlenken einer Stoßwelle, wobei eine Stoßwelle, die aus einzelnen Stoßwellenanteilen besteht, durch Beschleunigung eines Projektils auf einen Applikator, der eine Applikationsfläche aufweist, erzeugt wird und von der Applikationsfläche in einer Stoßrichtung emittiert wird, und die Stoßwelle von der Reflexionsfläche eines Reflektors zumindest teilweise reflektiert und zumindest teilweise auf einen Fokuspunkt fokussiert wird, hat gegenüber dem Stand der Technik den Vorteil, dass der Fokuspunkt nicht in einer Stoßrichtung liegt, wodurch nur ein minimaler Anteil der primären Stoßwelle in das zu behandelnde Gewebe eindringt.

Nach einer vorteilhaften Ausgestaltung des erfindungsgemäßen Verfahrens ist die Stoßrichtung tangential an den Fokuspunkt angenähert.

Nach einer zusätzlichen vorteilhaften Ausgestaltung des erfindungsgemäßen Verfahrens ist zumindest für einen Teil der Stoßwellenanteile eine Strecke zwischen einem

Punkt der Applikationsfläche, von dem dieser Stoßwellenanteil emittiert wird, und einem Punkt auf der Reflexionsfläche, von dem er reflektiert wird, und dem Fokuspunkt gleich.

Nach einer zusätzlichen vorteilhaften Ausgestaltung des erfindungsgemäßen Verfahrens wird eine Vorrichtung, nach einem der Ansprüche 1 bis 7, verwendet.

Weitere Vorteile und vorteilhafte Ausgestaltung der Erfindung sind der nachfolgenden Beschreibung, den Ansprüchen und den Zeichnungen entnehmbar.

### Zeichnung

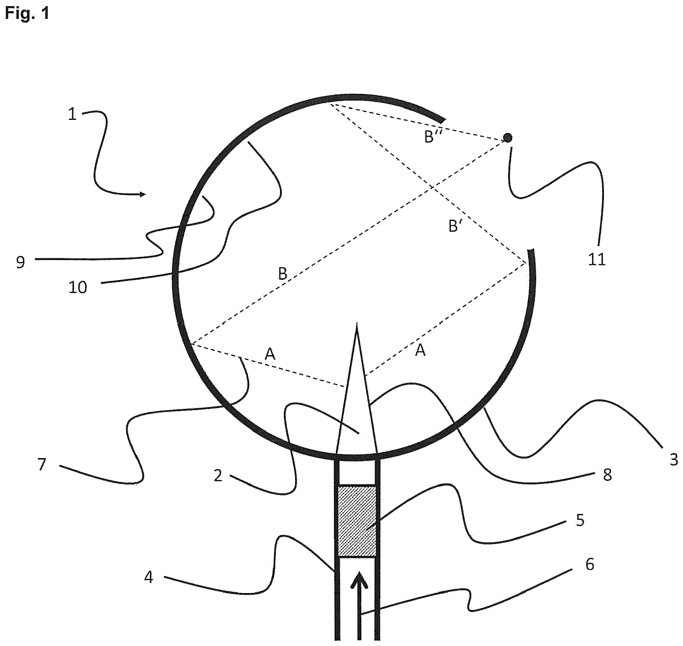

Ein bevorzugtes Ausführungsbeispiel des erfindungsgemäßen Gegenstands ist in der Zeichnung dargestellt und wird im Folgenden näher erläutert. Es zeigt
- Fig. 1: eine schematische Darstellung einer erfindungsgemäßen Vorrichtung.

### Beschreibung des Ausführungsbeispiels

Fig. 1 zeigt eine schematische Darstellung einer erfindungsgemäßen Vorrichtung 1 zur Umlenkung und Fokussierung von Stoßwellen. Die Vorrichtung umfasst einen Applikator 2 und einen Reflektor 3, die mit einem Projektil-Lauf 4 derart verbunden sind, dass ein im Projektil-Lauf 4 beschleunigtes Projektil 5 auf den Applikator 2 beschleunigt werden kann und mit diesem zusammenstößt. Der Stoß auf den Applikator 2 erfolgt in der Stoßrichtung 6 des Projektils 5 und löst Stoßwellen, die sich auf unterschiedlichen Stoßwellenanteilen 7 zusammensetzen aus, wobei einzelne Stoßwellenanteile 7 mit eigener Stoßrichtung von den einzelnen Punkten der Applikationsflächen 8 emittiert werden. Die Applikationsflächen 8 sind auf Reflexionsflächen 9 ausgerichtet und die jeweiligen Stoßwellenanteile 7 treffen in jeweiligen Reflexionspunkten 10 auf diesen auf. An den Reflexionspunkten 10 wird der Stoßwellenanteil 7 reflektiert und, sofern es sich nicht um eine Totalreflexion handelt, umgelenkt. Durch die spezifische, vorab berechnete, Struktur der Reflexionsflächen 9 erfolgt eine Fokussierung der Stoßwellenanteile 7 auf den Fokuspunkt 11, wobei durch die vorab Berechnung der Struktur der Reflexionsflächen 9 zumindest ein Teil der Stoßwellenanteile 7 die gleiche Strecke zurücklegt und diese Strecke sich aus den jeweils zu dem spezifischen Stoßwellenanteil 7 gehörigen Streckenanteilen A und B bzw. den Unterstreckenanteilen B' und B" zusammensetzt. Die Position des Fokuspunktes 11 ist primär abhängig von der Struktur der Reflexionsflächen 9 des Reflektors 3 und dem Applikator 2. Im dargestellten Ausführungsbeispiel ist der Reflektor 3 mit dem Projektil-Lauf 4 verbunden, wobei beispielsweise auch eine Verbindung über ein einhüllendes Gehäuse oder dergleichen möglich wäre. Der Reflektor 3 kann bei der erfindungsgemäßen Vorrichtung 1 einfach ausgetauscht werden und durch einen anderen Reflektor 3 ersetzt werden, der einen anderen Fokuspunkt 11 aufweist. Im Sinne der Erfindung ist der Fokuspunkt 11 nicht lediglich als Punkt mit einer im Idealfall infinitesimal kleinen Fläche zu verstehen, sondern kann auch eine Fokusfläche darstellen, um gleichzeitig einen größeren Bereich zu behandeln. Der Reflektor 3 kann zur besseren Stoßwellen-Vermittlung mit einem Übergangsmaterial gefüllt sein.

Alle in der Beschreibung dargestellten Merkmale können sowohl einzeln als auch in beliebiger Kombination miteinander erfindungswesentlich sein.

### Bezugszahlenliste

- 1: Vorrichtung
- 2: Applikator
- 3: Reflektor
- 4: Projektil-Lauf
- 5: Projektil
- 6: Stoßrichtung des Projektils
- 7: Stoßwellenanteil
- 8: Applikationsfläche
- 9: Reflexionsfläche
- 10: Reflexionspunkt
- 11: Fokuspunkt

## Patentansprüche

1. Vorrichtung (1) zur Umlenkung und Fokussierung von Stoßwellen,
- mit mindestens einem Applikator (2), wobei mindestens ein Applikator (2) mindestens eine Applikationsfläche (8) aufweist und von mindestens einer Applikationsfläche (8) in einer Stoßrichtung eine Stoßwelle emittiert werden kann,
- mit mindestens einem Reflektor (3), wobei mindestens ein Reflektor (3) mindestens eine Reflexionsfläche (9) und einen Fokuspunkt (11), auf den Stoßwellen durch die Reflexionsflächen (9) fokussiert werden können, aufweist, **dadurch gekennzeichnet,**
**dass** der Fokuspunkt (11) nicht in einer Stoßrichtung liegt.

2. Vorrichtung (1), nach Anspruch 1,
**dadurch gekennzeichnet,**
**dass** mindestens ein Applikator (2) und mindestens ein Reflektor (3) reversibel miteinander verbunden sind.

3. Vorrichtung (1), nach Anspruch 1 oder Anspruch 2,
**dadurch gekennzeichnet,**
**dass** der mindestens eine Reflektor (3) in Form einer Kugel geformt ist.

4. Vorrichtung (1), nach einem der vorhergehenden Ansprüche,
**dadurch gekennzeichnet,**
**dass** die mindestens eine Reflexionsfläche (9) des mindestens einen Reflektors (3) derart geformt ist, dass zumindest für einen Teil der Stoßwelle bzw. einen Stoßwellenanteil (7) eine Strecke, die sich als Summe aus einem Streckenanteil A und einem Streckenanteil B ergibt, gleich ist,
- wobei der Streckenanteil A die Strecke zwischen einem Punkt der Applikationsfläche (8), von dem dieser Stoßwellenanteil emittiert wird, und einem Punkt (10) auf der Reflexionsfläche (9), von dem er anschließend reflektiert wird, umfasst, und
- der Streckenanteil B die Strecke zwischen dem Punkt (10) auf der Reflexionsfläche (9), von dem dieser Stoßwellenanteil (7) reflektiert wird, und dem Fokuspunkt (11) umfasst.

5. Vorrichtung (1), nach einem der vorhergehenden Ansprüche,
wobei die Vorrichtung (1) mindestens ein Projektil (5) aufweist,
**dadurch gekennzeichnet,**
**dass** mindestens ein Projektil (5) aus einer Keramik gefertigt ist und/oder mindestens ein Applikator (2) aus einem Metall und/oder einer Legierung gefertigt ist.

6. Vorrichtung (1), nach Anspruch 5,
**dadurch gekennzeichnet,**
**dass** mindestens ein Applikator (2) aus einer Magnesium- und/oder Aluminiumlegierung gefertigt ist.

7. Vorrichtung (1), nach einem der vorhergehenden Ansprüche,
**dadurch gekennzeichnet,**
**dass** mindestens ein Applikator (2) eine kegelförmige Spitze aufweist.

8. Verwendung einer Vorrichtung (1) zur Umlenkung und Fokussierung von Stoßwellen, nach einem der vorhergehenden Ansprüche,
wobei die Stoßwellen zur Schmerztherapie eines Patienten erzeugt werden, **dadurch gekennzeichnet,**
**dass** die Intensität der Stoßwellen im Fokuspunkt (11) mittels eines Elektroenzephalogramms ermittelt wird.

9. Verwendung, nach Anspruch 8,
**dadurch gekennzeichnet,**
**dass** die Intensität der Stoßwellen entsprechend eines Signals im Elektroenzephalogramm angepasst wird.

10. Verfahren zum Fokussieren und Umlenken einer Stoßwelle,
- wobei eine Stoßwelle, die aus einzelnen Stoßwellenanteilen (7) besteht, durch Beschleunigung eines Projektils (5) auf einen Applikator (2), der eine Applikationsfläche (8) aufweist, erzeugt wird und von der Applikationsfläche (8) in einer Stoßrichtung emittiert wird,
- die Stoßwelle von der Reflexionsfläche (9) eines Reflektors (3) zumindest teilweise reflektiert wird und zumindest teilweise auf einen Fokuspunkt (11) fokussiert wird,
**dadurch gekennzeichnet,**
**dass** der Fokuspunkt (11) nicht in einer Stoßrichtung liegt.

11. Verfahren, nach Anspruch 10,
**dadurch gekennzeichnet,**
**dass** die Stoßrichtung tangential an den Fokuspunkt (11) angenähert ist.

12. Verfahren, nach Anspruch 10 oder Anspruch 11,
**dadurch gekennzeichnet,**
**dass** zumindest für einen Teil der Stoßwellenanteile (7) eine Strecke zwischen einem Punkt der Applikationsfläche (8), von dem dieser Stoßwellenanteil emittiert wird, und einem Punkt (10) auf der Reflexionsfläche (9), von dem er reflektiert wird, und dem Fokuspunkt (11) gleich ist.

13. Verfahren, nach einem der Ansprüche 10 bis 12,
**dadurch gekennzeichnet,**
**dass** eine Vorrichtung (1), nach einem der Ansprüche 1 bis 7, verwendet wird.
